Europäisches Patentamt

European Patent Office

Office européen des brevets

(19)

(11) Publication number: **0 015 665**
**B1**

(12) **EUROPEAN PATENT SPECIFICATION**

(45) Date of publication of patent specification: **01.09.82**

(51) Int. Cl.³: **C 07 C 21/06, C 07 C 17/34**

(21) Application number: **80300419.1**

(22) Date of filing: **13.02.80**

(54) Manufacture of vinyl chloride.

(30) Priority: **02.03.79 GB 7907386**

(43) Date of publication of application:
**17.09.80 Bulletin 80/19**

(45) Publication of the grant of the patent:
**01.09.82 Bulletin 82/35**

(84) Designated Contracting States:
**BE DE FR GB IT NL**

(56) References cited:
**GB - A - 1 096 847**
**GB - A - 1 281 278**
**GB - A - 1 442 117**
**JP - A - 52 031 005**

(73) Proprietor: **IMPERIAL CHEMICAL INDUSTRIES PLC**
**Imperial Chemical House Millbank**
**London SW1P 3JF (GB)**

(72) Inventor: **Allen, Christopher Storm**
**65 Sheraton Park**
**Stockton-on-Tees,Cleveland (GB)**

(74) Representative: **Roberts, John Stanley et al,**
**IMPERIAL CHEMICAL INDUSTRIES PLC Legal**
**Department: Patents Thames House North**
**Millbank**
**London SW1P 4QG (GB)**

Courier Press, Leamington Spa, England.

**0 015 665**

## Manufacture of vinyl chloride

This invention relates to a process for the manufacture of vinyl chloride by the hydrodechlorination of 1,1,2-trichloroethane and to catalysts for use therein.

In Japanese Patent Publication No. 77/31005 there is described a process for the manufacture of vinyl chloride by reacting 1,1,2-trichloroethane with hydrogen in the presence of a supported catalyst consisting of palladium chloride, copper chloride and an alkali metal chloride; however in this process the conversion of 1,1,2-trichloroethane to vinyl chloride (i.e. the number of moles of vinyl chloride produced per mole of 1,1,2-trichloroethane fed) is below 60%.

We have now found that relatively high conversions to vinyl chloride may be obtained by use of a catalyst comprising (a) a chloride of a noble metal from the group consisting of palladium, rhodium and platinum, (b) an alkali metal chloride and additionally (c) a chloride of iron. The said catalyst also has a long active life.

Thus according to the present invention there is provided a process for the manufacture of vinyl chloride by reacting 1,1,2-trichloroethane with hydrogen in the vapour phase at elevated temperature in the presence of a supported catalyst comprising a chloride of a noble metal and an alkali metal chloride, characterized in that the process is carried out at a temperature in the range from 200°C to 350°C in the presence of a catalyst comprising a chloride of a noble metal from the group consisting of palladium, rhodium and platinum and also containing a chloride of iron.

The chlorides of palladium and rhodium are especially preferred as the noble metal chlorides. Mixtures of the chlorides of two or more of the said noble metals may be used if desired.

The preferred alkali-metal chlorides are potassium chloride and cesium chloride; mixtures of two or more alkali-metal chlorides may be used if desired. Other components which may optionally be included in the catalyst are chlorides of copper and/or chlorides of rare-earth metals (for example cerium chloride).

The defined chlorides may be introduced as such during the preparation of the catalyst; alternatively, the metals may be introduced in the form of salts which are at least partially converted into the corresponding chlorides under the conditions of preparation or use of the catalyst.

The proportion of iron in the catalyst is preferably from 0.5 to 20 gram atoms per gram atom of noble metal, for example from 1 to 10 gram atoms of iron per gram atom of noble metal. When the catalyst also contains copper chloride, the preferred proportion of iron is towards the lower end of the said range; thus, for example, the catalyst may contain from 2 to 10 gram atoms of copper and from 0.5 to 5 gram atoms of iron per gram atom of noble metal.

The proportion of alkali metal is preferably from 1 to 25 gram atoms per gram atom of noble metal, for example from 2 to 12 gram atoms of alkali metal per gram atom of noble metal. When the catalyst also contains a rare-earth metal chloride, the preferred proportion of alkali metal is towards the lower end of the said range; thus, for example, the catalyst may contain from 2 to 10 gram atoms of rare-earth (for example cerium) and from 2 to 10 gram atoms of alkali metal per gram atom of noble metal.

The total proportion of metal chlorides is preferably at least 2% by weight (for example from 2% to 15%, especially from 5% to 10%) of the total weight of metal chlorides and support.

The metal chlorides may be supported upon conventional carrier materials, for example silica or alumina. Suitable carriers include aluminas having surface areas in the range from $10 \, m^2/g$ to $150 \, m^2/g$.

The catalyst may be employed in fixed, moving or fluidised beds.

The preferred reaction temperature is dependent to some extent upon the composition of the catalyst. With a catalyst composition containing iron but containing no copper (or containing less than 2 gram atoms of copper per gram atom of noble metal) the preferred temperatures are in the range from 300°C. With a catalyst containing 2 or more gram atoms of copper per gram atom of noble metal the reaction temperatures are somewhat lower, preferably in the range from 250°C to 300°.

Preferably there is employed at least the stoichiometric amount of hydrogen, that is, at least one mole of hydrogen per mole of 1,1,2-trichloroethane. Excess hydrogen, for example, up to two moles of hydrogen per mole of 1,1,2-tri-chloroethane may be employed but higher proportions of hydrogen are undesirable since there is increasing formation of ethylene as the proportion of hydrogen is increased.

Atmospheric, subatmospheric or superatmospheric pressures may be employed. Contact times are conveniently in the range from 2 to 30 seconds.

The 1,1,2-trichloroethane may be introduced into the reaction system, for example, by passing it into a flash vaporiser together with a diluent gas such as nitrogen which acts as a carrier gas. Alternatively hydrogen may be used both as reactant and carrier gas.

Vinyl chloride and hydrogen chloride formed in the process may be recovered by conventional means. For example the reaction product may be cooled to condense the organic product while hydrogen chloride passes through the condensing system. The organic product can be fractionally distilled to give pure vinyl chloride.

The following Examples illustrate the invention:

2

## 0 015 665

### Example 1

Catalyst (A) was made as follows:

The support for the catalyst was a gamma alumina of approximate surface area 60 m²/g and was in the form of cylindrical pellets of length 0.32 cm. To a weak solution of hydrochloric acid were added:

(i) 0.5 g palladous chloride
(ii) 1.9 g cupric chloride dihydrate
(iii) 0.45 g ferric chloride and
(iv) 4.7 g cesium chloride

100 g of the support were added to the solution containing the metal chlorides. The mixture was stirred continuously at 80°C to 120°C for a period of approximately 4 hours by which time the catalyst (A) was dry. This catalyst was for use in a fixed bed process.

Other supported catalysts (B) and (C) were prepared from metal chlorides as shown in Table 1 by a similar procedure except that the gamma alumina was replaced by alpha alumina of surface area of approximately 35 m²/g and of particle size 150 to 250 microns. These two catalysts were for use in a fluid bed process.

In Table 1 "% total chlorides" is the proportion of total metal chloride impregnants expressed as a percentage by weight of total metal chloride plus carrier.

### TABLE 1

| Catalyst | Atomic Ratios of Metals | | | | | | | % total chlorides |
|---|---|---|---|---|---|---|---|---|
| | Pd | Rh | Fe | Cu | K | Cs | Ce | |
| (A) | 1 | nil | 1 | 4 | nil | 10 | nil | 6.7 |
| (B) | nil | 1 | 4 | nil | nil | 5 | 5 | 7.8 |
| (C) | 1 | nil | 4 | nil | 5 | nil | nil | 3.2 |

### Example 2

The apparatus comprised a vertical heat resistant glass tube 30 cms long and 2.5 cms internal diameter surrounded by an electric furnace. The tube contained 90 g of the supported catalyst occupying 18 cm of the height of the tube. 1,1,2-trichloroethane was vaporised by passing it to a heated glass vessel containing porcelain particles held at a temperature of approximately 180°C. Nitrogen was passed through the vessel in such proportion that the volume of nitrogen was twice the volume of the vaporised 1,1,2-trichloroethane. Hydrogen was also mixed with the 1,1,2-trichloroethane and the resulting gaseous mixture was passed over the fixed bed catalyst (A) and through catalysts (B) and (C) maintaining the latter two catalysts in the fluidised state. The molar ratio of hydrogen to 1,1,2-trichloroethane was 1:1 in all runs. The contact time in all runs was approximately 4 seconds. The reaction temperatures and results obtained were as shown in Table 2. The product was recovered and analysed by conventional means.

### TABLE 2

| Catalyst | Temp. °C | % 1,1,2-trichloroethane reacted | Conversion to specific products | | |
|---|---|---|---|---|---|
| | | | VC | Ethylene | DCE |
| (A) | 265 | 88 | 80 | 4.5 | 4.5 |
| (B) | 290 | 90 | 82 | Nil | 6 |
| (C) | 320 | 93 | 86 | 0.5 | 3 |

VC denotes vinyl chloride

DCE denotes dichloroethylenes

"Conversion" denotes moles of specific product per 100 moles of 1,1,2-trichloroethane fed.

**0015 665**

## Claims

1. A process for the manufacture of vinyl chloride by reacting 1,1,2-trichloroethane with hydrogen in the vapour phase in the presence of a supported catalyst comprising a chloride of a noble metal and a chloride of an alkali metal, characterized in that the process is carried out at a temperature in the range from 200°C to 350°C in the presence of a catalyst comprising a chloride of a noble metal from the group consisting of palladium, rhodium and platinum and also containing a chloride of iron.

2. A process according to Claim 1 characterized in that the proportion of iron in the catalyst is from 0.5 to 20 gram atoms of iron per gram atom of noble metal.

3. A process according to Claim 2 characterized in that the proportion of iron is from 1 to 10 gram atoms of iron per gram atom of noble metal.

4. A process according to any of the preceding claims characterized in that the proportion of alkali metal is from 1 to 25 gram atoms of alkali metal per gram atom of noble metal.

5. A process according to Claim 4 characterized in that the proportion of alkali metal is from 2 to 12 gram atoms of alkali metal per gram atom of noble metal.

6. A process according to any of the preceding claims characterized in that the catalyst also contains a chloride of copper.

7. A process according to Claim 6 characterized in that the catalyst contains from 2 to 10 gram atoms of copper and from 0.5 to 5 gram atoms of iron per gram atom of noble metal.

8. A process according to any of the preceding claims characterised in that the alkali metal is potassium or cesium.

9. A process according to any of the preceding claims characterized that the support material is alumina having a surface area in the range from $10^2$/g to 150 m²/g.

10. A process according to any of the preceding claims characterized in that the total proportion of metal chlorides is from 2% to 15%, preferably from 5% to 10%, by weight based on the total weight of metal chlorides and support.

## Revendications

1. Procédé de production du chlorure de vinyle par réaction du 1,1,2-trichloroéthane avec l'hydrogène en phase vapeur en présence d'un catalyseur sur support comprenant un chlorure d'un métal noble et un chlorure d'un métal alcalin, caractérisé en ce que le procédé est exécuté à une température de l'intervalle de 200 à 350°C en présence d'un catalyseur comprenant un chlorure d'un métal noble choisi dans le groupe consistant en le palladium, le rhodium et le platine et contenant aussi un chlorure de fer.

2. Procédé suivant la revendication 1, caractérisé en ce que la proportion de fer dans le catalyseur est de 0,5 à 20 atomes-grammes de fer par atome-gramme de métal noble.

3. Procédé suivant la revendication 2, caractérisé en ce que la proportion de fer est de 1 à 10 atomes-grammes de fer par atome-gramme de métal noble.

4. Procédé suivant l'une quelconque des revendications précédentes, caractérisé en ce que la proportion de métal alcalin est de 1 à 25 atomes-grammes de métal alcalin par atome-gramme de métal noble.

5. Procédé suivant la revendication 4, caractérisé en ce que la proportion de métal alcalin est de 2 à 12 atomes-grammes de métal alcalin par atome-gramme de métal noble.

6. Procédé suivant l'une quelconque des revendications précédentes, caractérisé en ce que le catalyseur contient aussi un chlorure de cuivre.

7. Procédé suivant la revendication 6, caractérisé en ce que le catalyseur contient 2 à 10 atomes-grammes de cuivre et 0,5 à 5 atomes-grammes de fer par atome-gramme de métal noble.

8. Procédé suivant l'une quelconque des revendications précédentes, caractérisé en ce que le métal alcalin est le potassium ou le césium.

9. Procédé suivant l'une quelconque des revendications précédentes, caractérisé en ce que la matière du support est l'alumine ayant une surface spécifique de l'intervalle de 10 à 150 m²/g.

10. Procédé suivant l'une quelconque des revendications précédentes, caractérisé en ce que la proportion totale de chlorures métalliques est de 2 à 15%, de préférence de 5 à 10%, en poids sur la base du poids total des chlorures métalliques et du support.

## Patentansprüche

1. Verfahren zur Herstellung von Vinylchlorid durch Umsetzung von 1,1,2-Trichloräthan mit Wasserstoff in der Gasphase und in Gegenwart eines Trägerkatalysators, der ein Chlorid eines Edelmetalls und ein Chlorid eines Alkalimetalls enthält, dadurch gekennzeichnet, daß das Verfahren bei einer Temperatur im Bereich von 200 bis 350°C in Gegenwart eines Katalysators ausgeführt wird, der ein Chlorid eines Edelmetalls aus der aus Palladium, Rhodium und Platin bestehenden Gruppe und auch ein Chlorid von Eisen enthält.

4

2. Verfahren nach Anspruch 1, dadurch gekennzeichnet, daß der Anteil des Eisens im Katalysator 0,5 bis 20 Grammatome Eisen je Grammatom Edelmetall beträgt.

3. Verfahren nach Anspruch 2, dadurch gekennzeichnet, daß der Anteil des Eisens 1 bis 10 Grammatome Eisen je Grammatom Edelmetall beträgt.

4. Verfahren nach einem der vorhergehenden Ansprüche, dadurch gekennzeichnet, daß der Anteil des Alkalimetalls 1 bis 25 Grammatome Alkalimetall je Grammatom Edelmetall beträgt.

5. Verfahren nach Anspruch 4, dadurch gekennzeichnet, daß der Anteil des Alkalimetalls 2 bis 12 Grammatome Alkalimetall je Grammatom Edelmetall beträgt.

6. Verfahren nach einem der vorhergenden Ansprüche, dadurch gekennzeichnet, daß der Katalysator auch ein Chlorid von Kupfer enthält.

7. Verfahren nach Anspruch 6, dadurch gekennzeichnet, daß der Katalysator 2 bis 10 Grammatome Kupfer und 0,5 bis 5 Grammatome Eisen je Grammatom Edelmetall enthält.

8. Verfahren nach einem der vorhergenden Ansprüche, dadurch gekennzeichnet, daß das Alkalimetall aus Kalium oder Cäsium besteht.

9. Verfahren nach einem der vorhergehenden Ansprüche, dadurch gekennzeichnet, daß das Trägermaterial aus Aluminiumoxid mit einer Oberfläche im Bereich von 10 $m^2$/g bis 150 $m^2$/g besteht.

10. Verfahren nach einem der vorhergehenden Ansprüche, dadurch gekennzeichnet, daß der gesamte Anteil der Metallchloride 2 bis 15 Gew.-%, vorzugsweise 5 bis 10 Gew.-%, bezogen auf das Gesamtgewicht aus Metallchloriden und Träger, beträgt.